# DEMANDE DE BREVET EUROPEEN

(11) **EP 4 046 484 A1**
(43) Date de publication de la demande: **24.08.2022**
(21) Numéro de dépôt: 22154398.6
(22) Date de dépôt: 01.02.2022
(51) Int. Cl.: A01K 11/00, A01K 29/00, A61B 5/01, A61B 5/00, G16H 50/20

(54) **DISPOSITIF DE SUIVI EN TEMPS REEL D'ANIMAUX D'ELEVAGE**

(30) Priorité: 23.02.2021 FR 2101742
(71) Demandeur: Livestock Technology, 63000 Clermont Ferrand (FR)
(72) Inventeur: CLAIREAU, Thierry, 10310 Bangkok (TH); GUEROU, Etienne, 63000 Clermont Ferrand (FR)
(74) Mandataire: Dennemeyer & Associates S.A.

(57) **Abrégé**

Le dispositif de suivi en temps réel d'animaux (1) d'élevage comportant au moins un organe de collecte de données (2) équipé d'au moins un capteur d'une donnée physiologique ou physico-chimique, d'un moyen de communication et de transmission des données collectées par ledit capteur à au moins un organe fixe (10) de transmission et de réception de données relié à au moins un module distant (18) de traitement des données collectées et à un module de commande (16), caractérisé en ce que chaque organe de collecte de données (2) est équipé, de manière non amovible, d'un élément de stockage de données d'identification unique de type RFID (Radio Frequency IDentification), d'au moins un capteur d'une température corporelle en tant que donnée physiologique, d'au moins un moyen de communication par UWB (Ultra Wide Band) avec au moins trois organe fixes (10) de transmission et de réception de données constitutifs d'un moyen de localisation tridimensionnelle de l'organe (2) de collecte de données dans un espace délimité et en ce que chaque organe fixe (10) de transmission est pourvu d'au moins un capteur (14) d'une température ambiante, en tant que donnée physico-chimique, dans l'espace tridimensionnel délimité dans lequel se trouve l'organe (2) de collecte de données.

## Description

[La présente invention concerne un dispositif de suivi en temps réel d'animaux d'élevage.

L'expression « animaux d'élevage » regroupe des mammifères tels que des bovins, ovins, caprins, équins, porcins, mais également d'autres animaux tels que des oiseaux ou des volailles, des poissons ou des coquillages. Le terme élevage désigne l'élevage des animaux pratiqué sur terre ou en mer, dans des bâtiments ou espaces d'élevage, fermés ou en plein air, dans des parcs aquacoles ou conchylicoles, quel que soit le type de production concernée, par exemple pour la chair, le lait, les œufs, la reproduction ou pour d'autres buts. Par ailleurs, il peut s'agir d'animaux dits domestiques ou sauvages, l'élevage étant le fait que l'homme gère, à son profit, la vie d'un ou plusieurs animaux.

Du fait de la taille de certaines structures d'élevage qui regroupent plusieurs centaines ou plusieurs milliers d'animaux selon l'espèce concernée, cela implique un suivi sanitaire et économique constant afin d'optimiser la production tout en préservant la santé, animale et humaine. Il est donc nécessaire de collecter de nombreux paramètres pour chaque animal élevé, ces paramètres variant selon l'espèce et/ou le type de production concernée. A titre d'exemples non limitatifs, on peut citer comme paramètres l'identification et/ou le pédigrée de l'animal, sa localisation dans la structure d'élevage, des paramètres physiologiques relatifs à l'animal comme la température, la période de chaleur, le poids de l'animal ou autre. Pour collecter ces paramètres, on connait divers dispositifs utilisant des moyens électroniques de collecte, de stockage, de transmission des données. Ces moyens permettent également une utilisation automatique, suite à l'identification de l'animal, de machines telles que des distributeurs automatiques de concentrés (DAC), des distributeurs automatiques de lait (DAL), des automates de pesée, des robots de traite ou encore des compteurs à lait. Ces moyens sont généralement utilisés dans des élevages bovins de production laitière.

Parmi ces moyens électroniques on connait par WO-A-2012/125266 une méthode de suivi de bovins dans le cas d'une exploitation laitière. Des moyens d'identification sont positionnés dans une étable et permettent de suivre le animaux équipés d'étiquettes RFID. Le positionnement des animaux associé à des algorithmes permet de suivre la consommation en eau et en nourriture en modélisant les déplacements à proximité des sources d'eau et de nourriture. De même, en se basant sur les déplacements des animaux, on peut définir un index d'œstrus et un index de santé. US-A-2018/325382 concerne une boucle d'oreille pour bovins pourvue d'un capteur de température et d'un capteur de la température ambiante. Les données sont collectées et transmises à un centre de gestion. On connait également par US-A-2020/0296936, un capteur de position d'un bovin placé dans la boucle d'identification de l'animal. En fonction de la position de l'animal, on induit un déplacement de l'animal vers une zone donnée. US-A-2018/0303063 divulgue une solution de traçage de l'animal par une balise GPS installée dans la boucle d'identification de l'animal. US-A-2019130728 décrit une solution de collecte de données qui, via une étiquette RFID, sont transmises à des bornes placées en divers lieux sur l'exploitation, le propriétaire ayant accès aux données via l'informatique en nuage ou cloud. D'autres dispositifs, tel celui décrit par WO-A-2020129056, assurent la détection de l'animal à un endroit donné et ouvrent ou ferment des portes, pour gérer le déplacement de l'animal. WO-A-2020185255 utilise le Bluetooth et le Wi-Fi pour vérifier si l'animal est dans une zone précise qui lui est affectée. AU-A-2020204591 divulgue une capsule avalée par un ruminant et permettant la collecte et la transmission de données physiologiques relatives à la rumination. EP-B-3071023 concerne un collier incorporant un radar UWB permettant la collecte d'informations sur les paramètres physiologiques de l'animal, le dispositif comportant des moyens d'émission et de réception distincts. On connait aussi un système commercialisé sous le nom de SMARTBOW^{®} par la société ZOETIS pour la surveillance des vaches laitières. Ce système utilise des capteurs introduits dans la boucle d'identification de l'animal et collectant des mouvements de l'oreille caractéristiques de la rumination, des chaleurs, ainsi que la localisation de la vache, afin d'émettre des alertes relatives à l'état de l'animal, cela à partir de données collectées par des bornes installées dans les étables. La société AVERY DENNISON commercialise également, sous le nom de SMARTRAC, une étiquette RFID implantée dans la boucle d'identification ou sur l'animal et qui fournit toutes les informations d'identification et d'origine de l'animal, y compris les données relatifs à ses traitements médicaux.

Il existe donc de nombreuses solutions basées sur l'électronique et l'informatique permettant à un propriétaire de suivre ses animaux et d'optimiser la gestion de ceux-ci, cela avec un minimum de déplacements, de l'animal et/ou du propriétaire. Si de telles solutions sont utilisables pour de nombreuses espèces quel que soit le nombre d'animaux concernés, il n'en demeure pas moins que les informations collectées et traitées sont incomplètes et/ou spécifiques à une espèce. Les dispositifs existant sont généralement adaptés à une utilisation en extérieur et non pour des espaces clos et fermés tels des bâtiments d'élevage. De plus, pour l'élevage de certaines espèces, par exemple des porcins ou des volailles, avec plusieurs milliers d'individus regroupés dans un espace limité, même s'il n'est pas clos, les systèmes existants ont des performances relativement limitées, tant pour le type de données collectées que dans la collecte et le traitement en soi des données, tout en étant souvent dédiés à une espèce animale et/ou à des paramètres donnés.

.C'est à ce besoin que se propose de répondre l'invention en offrant un dispositif de suivi en temps réel des animaux d'élevage, utilisable quel que soit le nombre d'animaux et/ou l'espèce, propre à collecter et traiter de manière évolutive diverses données, cela dans un espace clos et fermé ou pour le moins tridimensionnel et délimité.

A cet effet, l'invention a pour objet un dispositif de suivi en temps réel d'animaux d'élevage comportant au moins un organe de collecte de données adapté pour équiper un animal, ledit organe étant équipé d'au moins un capteur d'une donnée physiologique ou physico-chimique, d'un moyen de communication et de transmission des données collectées par ledit capteur à au moins un organe fixe de transmission et de réception de données relié à au moins un module distant de traitement des données collectées et à un module de commande, caractérisé en ce que chaque organe de collecte de données est équipé, de manière non amovible, d'un élément de stockage de données d'identification unique de type RFID (Radio Frequency IDentification), d'au moins un capteur d'une température corporelle en tant que donnée physiologique de l'animal équipé de l'organe de collecte de données, d'au moins un moyen de communication par UWB (Ultra Wide Band) avec au moins trois organes fixes de transmission et de réception de données constitutifs d'un moyen de localisation tridimensionnelle de l'organe de collecte de données dans un espace tridimensionnel délimité et en ce que chaque organe fixe de transmission est pourvu d'au moins un capteur d'une température ambiante, en tant que donnée physico-chimique, dans l'espace tridimensionnel délimité dans lequel se trouve l'organe de collecte de données.

Ainsi, grâce à l'invention on dispose d'un moyen de suivi en temps réel des animaux qui permet de suivre plusieurs animaux, quel que soit leur nombre, leur espèce et/ou l'endroit où ils se trouvent, en intérieur dans un bâtiment d'élevage ou en extérieur mais dans tous les cas dans un espace tridimensionnel délimité, cela en intégrant à la fois des données concernant chaque animal et des données concernant l'environnement de chaque animal. L'utilisation de la localisation dans les trois dimensions au moins par UWB assure une précision élevée de localisation de l'organe de collecte de données, donc de facto de l'animal lorsque ce dernier est équipé de l'organe de collecte de données. Par ailleurs, les données d'identification unique assurent un suivi personnalisé et sécurisé de chaque animal. Ces données sont collectées, traitées et permettent à un utilisateur d'agir sur les conditions d'élevage et/ou sur l'animal, si besoin sans avoir à être présent sur le lieu d'élevage.

Selon des aspects avantageux mais non obligatoires de l'invention, un tel dispositif peut comprendre une ou plusieurs des caractéristiques suivantes :

le dispositif comprend au moins un organe dit actif agissant sur l'animal.

Le dispositif comprend au moins un organe dit actif agissant sur l'espace tridimensionnel.

Le module distant de traitement des données est hébergé dans le cloud ou informatique en nuage.

Le module distant de traitement de données est associé à un module d'apprentissage.

Le module de commande est associé à un terminal de commande distant dudit module.

Le dispositif comporte un module de lecture communiquant avec au moins un organe fixe de de transmission et de réception de données.

Le module de lecture communique avec au moins un organe actif agissant sur l'animal ou sur l'espace tridimensionnel.

L'invention sera mieux comprise et d'autres avantages de celle-ci apparaitront plus clairement à la lecture de la description qui va suivre, donnée uniquement à titre d'exemple non limitatif et faite en référence aux dessins annexés dans lesquels:

[Fig.1] est une vue latérale d'un animal stylisé, ici un porc, équipé d'un organe de collecte de données conforme à un mode de réalisation de l'invention,

[Fig.2] est une vue de dessus, à plus grande échelle, de l'organe de collecte de données de la figure 1 et

[Fig.3] est une vue schématique de l'utilisation du dispositif, selon un procédé conforme à un mode de réalisation de l'invention.

L'invention est particulièrement adaptée à l'élevage d'animaux dans un espace tridimensionnel délimité, notamment un espace clos et fermé, typiquement un bâtiment d'élevage, étant entendu que l'invention est également utilisable en extérieur, donc pour un espace ouvert, pour autant que l'espace tridimensionnel soit défini. Dans le cas d'un espace fermé, il est fréquent qu'un bâtiment d'élevage abrite plusieurs centaines voire plusieurs milliers d'animaux. Ces derniers sont élevés soit pour leurs chairs soit pour leurs laits, leurs œufs, leurs fourrures ou autres. Les élevages actuels sont de plus en plus rationalisés et industrialisés, en ce sens que la production doit se faire avec un rendement optimal, en un temps minimal, en préservant la santé et le bien-être des animaux tout en respectant les diverses réglementations. La concentration de nombreux animaux dans un espace délimité, qui plus est lorsqu'il est fermé, génère des contraintes, sanitaires et environnementales entre autres, qui nécessitent un suivi régulier des animaux et une gestion quotidienne des conditions d'élevage, cela avec un personnel dont l'effectif est limité.

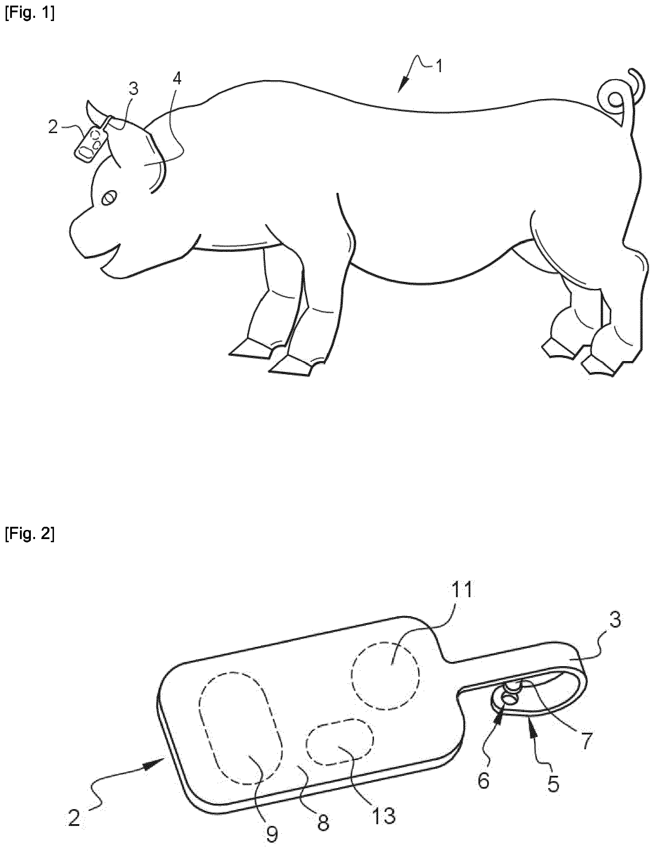

La figure 1 illustre un animal 1, en l'espèce un porc, équipé d'un organe de collecte de données 2, en l'espèce une boucle d'oreille. On conçoit aisément que l'invention peut s'appliquer à un bovin, un caprin, un ovin, un équin, une volaille, un poisson, un animal domestique ou non, donc à tout animal nécessitant un suivi dans une zone d'élevage, de reproduction, de vie, que cette zone soit ouverte ou fermée, en extérieur ou en intérieur pour autant que cette zone soit tridimensionnelle et délimitée. Ainsi à titre d'exemples non limitatifs, on peut citer des élevages de porcs, de bovins ou de volailles, des élevages de chevaux ou d'ovins en intensif ou en extensif ou en semi-liberté, les animaux n'étant regroupés dans des lieux donnés que pour certaines périodes, des bassins d'aquacultures en mer ou en eau douce, des parcs ou réserves pour animaux sauvages, des enclos d'élevage de gibier ou d'une manière générale toute zone délimitée dans laquelle se trouve un nombre important d'animaux nécessitant une gestion et un suivi. On conçoit alors que le dispositif peut, comme illustré, être une boucle fixée à l'oreille de l'animal mais également un collier, une bague ou une étiquette fixée sur le corps de l'animal. Dans tous les cas, la forme, le poids, les dimensions et/ou le matériau constitutif du dispositif sont adaptés à l'animal et à son environnement.

Ici, la boucle 2 comprend, dans le mode réalisation illustré, une patte de fixation 3 sur une oreille 4 du porc 1. Comme cela ressort de la figure 2. la fixation s'effectue, de manière connue en soi, par pincement de l'oreille entre deux parties constitutives de la boucle 2. A titre illustratif, à la figure 2, une extrémité libre 5 de la patte 3 est pourvue d'un orifice 6 d'insertion en force de l'extrémité libre d'une tige 7 fixée sur la patte 3. La liaison entre la tige 7 et l'extrémité 5 est effectuée de façon définitive, à la manière d'un rivet, la tige 7 passant par un perçage ménagé dans l'oreille de l'animal. En variante connue et non illustrée, d'autres modes de fixation sont prévus, par exemple, la boucle est en deux parties similaires placées de chaque côté de l'oreille 4 de l'animal 1 ou bien la patte est rigide et l'orifice est prévu sur une autre pièce positionnée sur l'autre face de l'oreille 4 de l'animal 1. Dans tous les cas, la tige est introduite dans un perçage réalisée dans l'oreille de l'animal et la fixation de la boucle 2 est définitive. Il n'est pas possible, accidentellement ou non, d'enlever la boucle de l'oreille de l'animal sans détruire la boucle ou sans blesser l'animal.

La partie 8 dite active de la boucle 2 est formée, dans l'exemple illustré, par une partie rectangulaire en un matériau rigide, insensible aux conditions environnementales et inerte vis-à-vis des ondes électromagnétiques. Il s'agit par exemple de polyuréthane thermoplastique ou TPU.

Typiquement, la partie 8 a des dimensions et une épaisseur suffisantes, entre 30 mm et 100 mm de long pour 25 mm à 50 mm de large et 3 mm à 18 mm d'épaisseur, dans le cas d'une utilisation sur des porcs, pour permettre l'insertion dans le matériau de divers éléments d'identification, de localisation et de collecte de paramètres physiologiques ou physico-chimiques. En particulier, selon l'invention, la partie 8 comprend un moyen de communication et de transmission de données ou antenne 9. Un tel moyen utilise au moins la technologie UWB (Ultra Wide Band). Il s'agit d'une technologie sans fil qui utilise un large spectre de fréquences, à savoir une largeur de bandes d'au moins 250 MHz, voire d'au moins 500MHZ selon l'organisme de régulation concerné. Ici, la largeur de bandes est comprise entre 3,1GHz et 10 GHz. La technologie UWB a une faible consommation d'énergie et une fiabilité élevée. Outre une utilisation pour la transmission de données, l'UWB est utilisé pour positionner et suivre des objets en temps réel. Les systèmes de positionnement par UWB offrent une précision d'au moins 30 cm voire dans certains cas entre 5 cm et 10 cm, autant à l'intérieur d'un bâtiment qu'à l'extérieur et cela en deux ou trois dimensions. Il est ainsi possible de connaitre et suivre la position de la boucle 2, donc de facto de l'animal 1 équipé de la boucle 2, dans un espace délimité, fermé ou non, cela par rapport à une surface, par exemple le sol d'un bâtiment d'élevage mais aussi par rapport au volume de l'espace, donc savoir si la boucle est sur le sol ou en hauteur dans le bâtiment d'élevage. Une telle possibilité est adaptée aux bâtiments dans lesquels l'élevage s'effectue sur plusieurs niveaux. Par ailleurs, la transmission par UWB est peu ou pas sensible aux obstacles. De ce fait, les matériaux constitutifs du bâtiment ou présent dans l'espace d'élevage n'affectent pas ou peu la transmission de données et les communications, donc la localisation de la boucle 2. Dans un autre mode de réalisation, le moyen de communication et de transmission de données utilise, en complément de la technologie UWB, la technologie BLE (Bluetooth Low Emission) dont les fréquences sont comprises entre 2,4 GHz et 2, 483 GHz. La technologie BLE offre une faible consommation d'énergie ainsi qu'une facilité de mise en œuvre. Par ailleurs, l'utilisation du Bluetooth permet l'emploi de smartphones comme moyen de lecture. Néanmoins, la précision de localisation est très inférieure à celle obtenue par UWB, ce qui limite son emploi à des configurations où la localisation précise des animaux n'est pas nécessaire et/ou la dynamique de déplacement des animaux n'est pas prise en compte. En complément, on peut également associer, pour une identification dite rapprochée avec un lecteur placé à quelques centimètres de la boucle, la technologie NFC. Cette technologie est employée comme interface pour reprogrammer certains éléments de la boucle et/ou pour recharger des organes de stockage d'énergie, typiquement des batteries implantées dans la boucle.

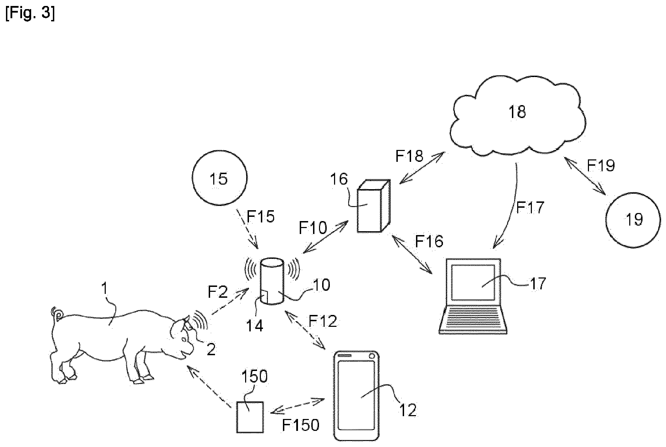

Pour effectuer la localisation du moyen 9, dans un espace délimité, fermé ou non, on définit la position du moyen 9 par rapport à une référence géographique connue et fixe. Dans le cas présent, pour une localisation en trois dimensions, selon l'invention, il faut au moins trois points de références fixes. Ici le terme fixe doit être compris comme l'absence de mouvement du point de référence lors de la mesure. Une fois la mesure ou la série de mesures faite(s), il est possible de déplacer les points de référence en un autre endroit, par exemple un autre bâtiment d'élevage lorsque les animaux sont déplacés dans un nouveau bâtiment. En variante, les points sont présents de manière définitive dans chacun des espaces qui sont ou seront occupés par les animaux. Ces points de référence, également nommés bornes, ancres ou balises évaluent la distance et la direction des moyens 9 par rapport auxdits points de référence, cela par mesure du temps de propagation des signaux, ce qui permet un calcul de la position des moyens 9 par trilatération. Un point de référence est, avantageusement, intégré à une borne 10, illustrée à la figure 3. Avantageusement, les bornes 10 sont positionnées dans un endroit où les animaux ne peuvent pas avoir un contact direct avec elles, afin d'éviter tout risque de dégradation des bornes par les animaux. Pour cela, elles sont, par exemple, placées en hauteur par rapport aux animaux. Dans un autre mode de réalisation, une borne 10 reçoit les données de plusieurs moyens 9, ceci afin de gérer simultanément plusieurs organes 2. En variante non illustrée, le point de référence est formé par un boitier dédié ou intégré dans un autre objet que la borne 10. Dans tous les cas, dans le cadre de l'invention, le point de référence a une position géographique connue. Ici, les bornes 10 sont réalisées en un matériau rigide, inerte aux conditions environnementales, résistant aux chocs, éventuellement aux morsures des animaux et inerte vis-à-vis des composants électroniques qu'elle reçoit. Typiquement une borne 10 est en PVC (polychlorure de vinyle) PU (polyuréthane) ou en acrylique et ses dimensions sont adaptées aux composants qu'elle reçoit et à l'espace dans lequel elle est placée.

La boucle 2 reçoit également un élément de stockage de données d'identification 11. Ici l'élément 11 est une étiquette de type RFID (radio Frequency IDentification). Une étiquette RFID permet de stocker des données d'identification sur une puce électronique associée à une antenne RFID qui permet l'échange de données avec un lecteur RFID dédié. La distance de lecture des étiquettes RFID varie en général entre un mètre et une quinzaine de mètres, selon le lecteur utilisé. Ici, le lecteur est soit un lecteur mobile 12, soit un lecteur fixe, avantageusement incorporé à la borne 10. Les donnée d'identification contenues dans l'étiquette RFID référencée 11 sont uniques et ne peuvent être modifiées. Elles sont donc spécifiques de l'animal 1 porteur de la boucle 2, pour autant bien entendu que la boucle 2 soit fixée sur l'animal 1. Afin de garantir le caractère unique et non réutilisable de l'étiquette RFID, cette dernière est positionnée dans une partie de la boucle qui sera détruite lors, par exemple, de la mort ou de l'abattage de l'animal. Ainsi, l'étiquette RFID est placée dans la patte 3 de la boucle, cette patte étant coupée lorsque l'animal est mort ou lors de son abattage.

La boucle 2 comprend également au moins un capteur 13 d'une donnée physiologique relative à l'animal 1 équipé de la boucle 2. Il s'agit d'un capteur d'une température corporelle de l'animal équipé de la boucle 2. Avantageusement il s'agit d'un capteur de température par contact avec la peau de l'animal au niveau de la zone de fixation de la boucle 2. On conçoit que la boucle 2 peut comporter d'autres capteurs destinés à collecter d'autres données physiologiques relatives à l'animal. Il peut s'agir, à titre d'exemples non limitatifs, du rythme cardiaque, du taux de graisse de l'animal mesuré par effet doppler, du taux d'oxygène dans le sang mesuré par oxymétrie, de détection des chaleurs, du début de lactation ou autre. Ces capteurs placés dans la boucle 2 peuvent être associés avec d'autres capteurs ou moyens de collecte de données placés ailleurs sur l'animal, par exemple autour du cou, de l'abdomen ou autre. Dans tous les cas, le capteur 13 et les autres capteurs sont adaptés à la donnée à mesurer et à collecter ainsi qu'à l'animal.

Les divers éléments présents dans la boucle 2, à savoir le moyen de communication 9, l'étiquette RFID 11 et le ou les capteurs(s) 13 sont disposés dans la masse de la boucle 2 afin d'être protégés de toute dégradation volontaire ou non, par exemple lorsque l'animal se frotte sur une surface dure, en cas de morsure et contre les conditions environnementales. Par ailleurs, les positions relatives de chaque élément 9, 11 et 13 sur la boucle 2 sont définies afin d'éviter toute interférence. En d'autres termes le calepinage des éléments 9, 11, 13 sur la boucle 2 est adapté aux nombres et/ou types d'éléments présents ainsi qu'à l'environnement dans lequel sera la boucle 2. Certains des éléments constitutifs de la boucle 2, lorsqu'il s'agit d'éléments actifs, par exemple des capteurs 13, nécessitent de l'énergie pour fonctionner. En conséquence, même si la durée de vie d'une boucle 2 équipée d'un moyen de stockage d'énergie, par exemple une pile de type pile bouton, est de plusieurs mois, il peut être nécessaire de recharger en électricité la boucle. Cela se fait, par exemple, par induction ou par une autre technique connue en soi. Dans un autre mode de réalisation, on utilise une capacité rechargeable par NFC.

Le dispositif comprend également au moins un capteur d'une donnée physico-chimique qui concerne l'espace tridimensionnel dans lequel évolue l'animal 1. En l'espèce, au moins un de ces capteurs est un capteur de température 14, par exemple placé sur la borne 10 ou bien en un autre point distant de la borne 10 mais, dans tous les cas, en un endroit représentatif d'une température ambiante de l'espace considéré. Des capteurs d'hygrométrie, de luminosité, de détection de gaz tel que CO2, NH3, NOx, CH4 ou d'autres gaz ou d'autres données physico-chimiques susceptibles d'affecter la santé ou le comportement des animaux ou des personnes présentes auprès des animaux ou encore l'espace tridimensionnel, peuvent équiper une ou plusieurs bornes ou bien être placés dans des zones dédiées, comme cela est illustré par la référence 15 à la figure 3.

De même, l'espace dans lequel se trouvent les animaux peut comporter des organes actifs sur les animaux et/ou sur les conditions environnementales présentes dans l'espace. Ces organes sont schématiquement représentés sous la référence 150. Par l'expression « organe actif », on désigne, par exemple, des distributeurs d'un traitement sanitaire ou médical, des plaques de pesée, des brumisateurs, des ventilateurs, des extracteurs d'air, des radiateurs, des lampes, des moyens d'ouverture et de fermeture de portes, de volets, de distributeurs de nourriture, de litière, des organes de nettoyage ou d'arrosage automatique, des robots de traite, de contention ou d'autres organes connus en soi. Dans tous les cas, les capteurs 15 et/ou les organes actifs 150 sont adaptés pour communiquer, en émission et/ou en réception, avec au moins le lecteur mobile 12. On conçoit que ce lecteur 12, par exemple une tablette, un smartphone ou un lecteur spécifique, permet de recevoir et transmettre des données et, de facto, de générer des instructions aux divers éléments avec lesquels il est en communication, à savoir les capteurs 15, les organes actifs 150 ou encore les éléments présents dans la borne 10.

Le dispositif comporte aussi au moins un module central de commande 16. Ce dernier est avantageusement placé dans une zone à laquelle les animaux n'ont pas un accès aisé. Ainsi, le module 16 est distant de l'espace tridimensionnel dans lequel se trouve les animaux, par exemple dans une zone dédiée à l'administration d'un ou plusieurs élevages, cette zone pouvant être éloignée de plusieurs kilomètres de l'espace d'élevage. La communication entre la borne 10 et le module de commande 16 s'effectue selon la double flèche F10 par un moyen filaire ou non filaire, par exemple par les réseaux 3G/4G/5G, LTU, Cat 5/6.

Le module de commande 16 est également en communication, filaire ou non, selon la double flèche F16 avec un terminal de commande 17. Le terminal 17 est, par exemple un ordinateur, une tablette. On conçoit que le terminal 17 peut se situé dans la même zone que le module 16 ou être distant de ce dernier de plusieurs kilomètres. Outre une communication avec le terminal 17, le module 16 échange également des données, à des fins de stockage et d'archivage, selon la double flèche F18 avec l'informatique en nuage ou cloud selon la terminologie anglaise et référencé 18 à la figure 3. Pour mémoire, le terme cloud désigne des services informatiques, par exemple de stockage, de mise en réseau, de fourniture d'applications informatiques, cela via le réseau Internet par un fournisseur d'accès au réseau internet. Le terminal 17 peut également, selon la flèche F17, récupérer des services informatiques et/ou des données stockées dans le cloud 18.

Selon un mode de réalisation illustré à la figure 3, le dispositif peut comporter également des modules d'apprentissage 19 qui, par échange de données selon la double flèche F19 avec le cloud 18 permettent d'enrichir les données et les services informatiques présent dans le cloud 18. Avantageusement, le module 19 met en œuvre des algorithmes du domaine de l'intelligence artificielle.

Le fonctionnement du dispositif est maintenant décrit en référence à la figure 3. Le dispositif objet de l'invention comprend au moins un organe de collecte de données 2 solidaire d'un animal 1, au moins trois points de référence ou bornes 10 disposés dans trois endroits d'un espace délimité et dédié à l'élevage d'au moins un animal, les coordonnées géographiques ainsi que l'altitude de ces endroits étant connus et invariables au moins le temps où l'animal est présent dans l'espace. Les bornes 10 sont donc immobiles au moins le temps du suivi de l'animal dans l'espace dédié. Chaque organe de collecte 2, ici une boucle d'oreille, communique avec chaque borne 10, selon la flèche F2, pour transmettre des données, par le moyen de communication 9 présent dans la boucle 2, donc par la technologie UWB et/ou BLE. En particulier, les données d'identification de l'animal contenues dans l'étiquette RFID 11 ainsi qu'au moins la température corporelle de l'animal 1 collectée par le capteur 13 sont envoyées à la borne 2 la plus proche de l'animal par UWB et/ou BLE, selon la flèche F2. Un tel échange de données peut se faire de manière automatique, par exemple à intervalles connus et réguliers mais aussi à la demande, sous l'action du lecteur mobile 12. En d'autres termes, selon les besoins, un utilisateur du dispositif collecte, à la demande, des données sur un ou plusieurs animaux présents dans un ou plusieurs espaces d'élevage dédiés. En effet, un même lecteur 12 ou smartphone peut communiquer, selon la double flèche F12, avec plusieurs jeux de bornes 10 ou bien avec plusieurs espaces d'élevage distants, si besoin en simultanée. Ainsi, une personne peut suivre et gérer de manière distante plusieurs centaines voire milliers d'animaux et/ou plusieurs espaces d'élevage. Le lecteur 12 peut également échanger des données avec des organes actifs 150 présents dans l'espace d'élevage, selon la double flèche F150. Ces organes actifs vont effectuer, sous l'action du lecteur 12, des actions relatives à un ou plusieurs animaux 1 et/ou à leurs environnements. Il s'agit, par exemple, de distribution de nourriture, de traitement sanitaire ou vétérinaire, d'ouvertures de portes, de mise en route d'une ventilation, d'une filtration de l'air, d'une brumisation, d'un éclairage, d'un chauffage, de distribution de nourriture ou d'eau, de mise en route d'un robot de traite, d'un pesage ou tout autre action agissant sur l'animal et/ou sur son environnement afin d'optimiser ses conditions d'élevage et/ou ses paramètres biologiques. Dans un autre mode de réalisation, un espace d'élevage accueille plusieurs espèces animales ou des animaux d'une même espèce mais à différents stades de développement. A titre d'exemple on peut citer des animaux adultes et des jeunes animaux élevés ensemble, des ovins et des caprins ensemble, des oies et des canards ensemble dans le même espace. Dans une telle configuration, chaque espèce animal ou chaque stade de développement donné d'un animal, par exemple un adulte et un jeune, est associé avec des bornes 10 et/ou des capteurs 15 et/ou des organes actifs 150 donnés mais présent dans un même espace.

Afin d'optimiser ses actions, le lecteur 12 reçoit indirectement, via les liaisons selon les double flèches F12 et F15, des données provenant de capteurs 15 et transitant par la borne 10. Les données collectées par le capteur 14 monté sur la borne 10 sont également utilisées. On conçoit que les données collectées sont variables, selon les types de capteurs et/ou selon le type d'animal élevé et/ou les conditions d'élevage. Néanmoins, les données concernant d'une part la température corporelle de l'animal prise par le capteur 13 et d'autre part la température ambiante dans l'espace tridimensionnel d'élevage collectée par le capteur 14 sont systématiquement prises en compte par le dispositif, cela quel que soient les autre données collectées par les divers capteurs.

Les données collectées, donc au moins les températures corporelle et ambiante, sont transmises par la borne 10 au module central de commande 16, selon la double flèche F10. Ce module 16, qui comprend un moyen de calcul, donc des algorithmes spécifiques à l'élevage et aux animaux concernés, d'une part transmets des données, via la liaison F18, dans le cloud 18, cela à des fins de stockage et, selon un mode de réalisation non obligatoire, à des fins d'apprentissage automatique, via un module 19 dédié. il est ainsi possible d'adapter en permanence le fonctionnement d'appareils automatiques concernant les conditions d'élevage aux besoins et/ou au comportement des animaux présents dans l'espace d'élevage.

Le terminal de commande 17, qui peut être distant ou non du module de commande 16, permet à au moins un utilisateur de prendre connaissance des données collectées et/ou stockées dans le cloud 18, d'induire des actions, cela via les liaisons F16 et F17 en agissant sur le module de commande 16. L'utilisateur peut également planifier des actions à venir sur les animaux, sur les conditions d'élevage ou sur la collecte et/ou la valorisation des produits d'élevage. A titre d'exemples on peut citer, la distribution de nourriture, de médicaments, des modifications de la température, de l'humidité dans l'espace d'élevage, le déclenchement de commande de produits, de matériels, l'initialisation du ramassage du produit fourni par les animaux tels que le lait ou les œufs ou des animaux eux-mêmes en vue de leur abattage ou de leur déplacement vers un autre espace d'élevage. Certaines actions peuvent être automatisées, par la mise en route de ventilateurs ou de brumisateurs en cas de franchissement d'une température de consigne dans le bâtiment d'élevage.

Dans tous les cas, chaque animal est, en permanence, suivi, tant au niveau de ses paramètres biologiques que de sa position dans l'espace d'élevage, cela grâce à une géolocalisation précise par au moins trois bornes 10. La technique utilisée permet une précision de localisation optimale qui être inférieure au mètre, cela selon les trois axes, quel que soit le nombre d'animaux présents dans l'espace.

L'invention permet de suivre et de gérer, pour une personne seule, en continu et à distance, un ou plusieurs espaces d'élevage, fermés ou ouverts, abritant plusieurs centaines d'animaux, cela avec une large variété de paramètres physico-chimiques ou biologiques, les paramètres étant aisément modifiables. Ainsi, le dispositif objet de l'invention peut être mis en œuvre dans un élevage de porcs, comme illustré, mais aussi un élevage d'ovins, de caprins, de bovins, de lapins, de volailles ou de poissons. Il est également possible de le mettre en œuvre pour de l'élevage d'animaux en parcs clos mais ouverts, tels que des parcs à ovins, caprins, bovins, équins ou pour des animaux sauvages ou du gibier.

## Revendications

1. [Dispositif de suivi en temps réel d'animaux (1) d'élevage comportant au moins un organe de collecte de données (2) adapté pour équiper un animal (1), ledit organe étant équipé d'au moins un capteur d'une donnée physiologique (13) ou physico-chimique, d'un moyen de communication et de transmission (9) des données collectées par ledit capteur (13) à au moins un organe fixe (10) de transmission et de réception de données relié à au moins un module distant (18) de traitement des données collectées et à un module de commande (16), **caractérisé en ce que** chaque organe de collecte de données (2) équipant un animal est équipé, de manière non amovible, d'un élément de stockage de données d'identification unique (11) de type RFID (Radio Frequency IDentification), d'au moins un capteur (13) d'une température corporelle en tant que donnée physiologique de l'animal (1) équipé de l'organe de collecte de données (2), d'au moins un moyen de communication (9) par UWB (Ultra Wide Band) avec au moins trois organes fixes (10) de transmission et de réception de données constitutifs d'un moyen de localisation tridimensionnelle de l'organe de collecte de données (2) dans un espace tridimensionnel délimité et **en ce que** chaque organe fixe (10) de transmission est pourvu d'au moins un capteur (14) d'une température ambiante, en tant que donnée physico-chimique, dans l'espace tridimensionnel délimité dans lequel se trouve l'organe de collecte de données (2).

2. Dispositif selon la revendication 1, **caractérisé en ce que** le dispositif comprend au moins un organe (150) dit actif agissant sur l'animal (1).

3. Dispositif selon la revendication 1, **caractérisé en ce que** le dispositif comprend au moins un organe dit actif agissant sur l'espace tridimensionnel.

4. Dispositif selon la revendication 1, **caractérisé en ce que** le module distant de traitement des données (18) est hébergé dans le cloud ou informatique en nuage.

5. Dispositif selon la revendication 4, **caractérisé en ce que** le module distant de traitement de données (18) est associé à un module d'apprentissage (19).

6. Dispositif selon la revendication 1, **caractérisé en ce que** le module de commande (16) est associé à un terminal de commande (17) distant dudit module.

7. Dispositif selon la revendication 1, **caractérisé en ce que** le dispositif comporte un module de lecture (12) communiquant avec au moins un organe fixe (10) de de transmission et de réception de données.

8. Dispositif selon la revendication 7, **caractérisé en ce que** le module de lecture (12) communique avec au moins un organe actif (150) agissant sur l'animal ou sur l'espace tridimensionnel.
